(19) Europäisches Patentamt
European Patent Office
Office européen des brevets

(11) Veröffentlichungsnummer: **0 002 223**
**B1**

(12) **EUROPÄISCHE PATENTSCHRIFT**

(45) Veröffentlichungstag der Patentschrift: **18.03.81**

(51) Int. Cl.³: **C 07 D 221/14,** D 06 L 3/12

(21) Anmeldenummer: **78101405.5**

(22) Anmeldetag: **18.11.78**

(54) **Naphthalimidderivate, Verfahren zu ihrer Herstellung, und ihre Verwendung als optische Aufheller.**

(30) Priorität: **29.11.77 DE 2753152**

(43) Veröffentlichungstag der Anmeldung:
**13.06.79 Patentblatt 79/12**

(45) Bekanntmachung des Hinweises auf die Patenterteilung:
**18.03.81 Patentblatt 81/11**

(84) Benannte Vertragsstaaten:
**CH DE FR GB**

(56) Entgegenhaltungen:
**FR-A-2 276 300**
**CHEMICAL ABSTRACTS, Vol. 81, Nr. 12, Zusammenfassung Nr. 65248d, 23.September 1974, Columbus, Ohio, USA,**
**IMABORI SEIICHI et al., »Fluorescent whitener«,**
**CHEMICAL ABSTRACTS, Vol. 81, Nr. 18, Zusammenfassung Nr. 107281s, 4. November 1974, Columbus, Ohio, USA**
**IMABORI SEIICHI et al., »Optical whitening agents«**
**CHEMICAL ABSTRACTS, Vol. 78, Nr. 12, Zusammenfassung Nr. 73568m, 26.März 1973, Columbus, Ohio, USA**
**OKADA HIROSHI et al., »Fluorescent whitening for polyesters fibers«**

(73) Patentinhaber: **BASF Aktiengesellschaft, Carl-Bosch-Strasse 38, D-6700 Ludwigshafen (DE)**

(72) Erfinder: **Karg, Jochen, Dr., Karlsbader Strasse 7, D-6700 Ludwigshafen (DE)**
Erfinder: **Patsch, Manfred, Dr., Fritz-Wendel-Strasse 4, D-6706 Wachenheim (DE)**
Erfinder: **Himmele, Walter, Dr., Eichenweg 14, D-6909 Walldorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



BUNDESDRUCKEREI BERLIN

## Naphthalimidderivate, Verfahren zu ihrer Herstellung und ihre Verwendung als optische Aufheller

Die Erfindung betrifft Verbindungen der allgemeinen Formel I

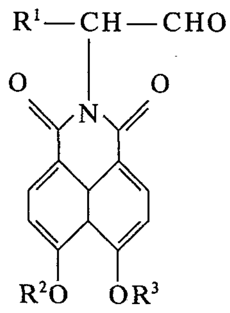

(I)

in der

$R^1$ $C_1$- bis $C_4$-Alkyl und
$R^2$ und $R^3$ unabhängig voneinander $C_1$- bis $C_4$-Alkyl, $\beta$-Hydroxyäthyl, $\beta$-Hydroxypropyl, $\beta$-$C_1$- bis $C_4$-Alkoxyäthyl oder $\beta$-$C_1$- bis $C_4$-Alkoxypropyl

sind.

$C_1$- bis $C_4$-Alkylreste für $R^1$, $R^2$ und $R^3$ sind beispielsweise n- oder i-Propyl, n- oder i-Butyl und vorzugsweise Methyl oder Äthyl.

Alkoxyalkylreste $R^2$ und $R^3$ sind beispielsweise

$C_2H_4OC_2H_5$, $C_2H_4OC_3H_7$, $C_2H_4OC_4H_9$,

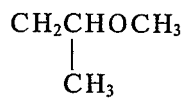

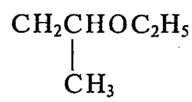

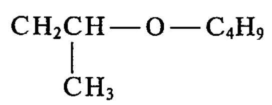

und vorzugsweise $C_2H_4OCH_3$.

Zur Herstellung der Verbindungen der Formel I kann man Verbindungen der Formel II

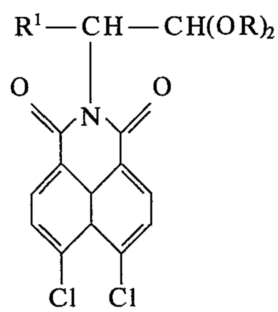

(II)

mit Verbindungen der Formel

$R^2OMe$ und/oder $R^3OMe$

umsetzen und anschließend hydrolysieren. Me ist dabei vorzugsweise ein Alkalimetall und R ist $C_1$- bis $C_4$-Alkyl. Die Verwendung von Mischungen von $R^2OMe$ und $R^3OMe$ ist bevorzugt.

Die Reaktionen sind im Prinzip bekannt, Einzelheiten können den Beispielen entnommen werden, in denen sich Angaben über Teile und Prozente, sofern nicht anders vermerkt, auf das Gewicht beziehen.

Die Verbindungen der Formel I eignen sich als optische Aufheller für synthetische Fasern, insbesondere für Polyester und Acetatfasern, auf denen man hohe Weißgrade mit geringen Mengen an Aufhellern erhält. Ferner sind die gute Lichtechtheit und die grünstichige Weißnuance hervorzuheben.

Einige der Verbindungen eignen sich auch für die Masseaufhellung.

Für $R^2$ und $R^3$ sind Methyl und Äthyl besonders bevorzugt. Weiterhin sind Verbindungen bevorzugt, bei denen $R^2$ und $R^3$ verschieden sind.

Gegenüber nächtvergleichbaren aus Chem. Abstracts, Vol. 78, Nr. 12, Zusammenfassung 73 568 m und Vol. 81, Nr. 18, Zusammenfassung 107 281 s bekannten Naphthalimidverbindungen zeichnen sich die erfindungsgemäßen Derivate durch eine bessere Aufhellwirkung von Polyesterfasern aus.

Beispiel 1

87 Teile 4,5-Dichlor-N-(1',1'-dimethoxypropyl-2')-naphthalimid, 630 Teile Methanol und 78 Teile Butyltriglykol werden vorgelegt. Unter Rühren werden bei 50 bis 60°C 146 Teile einer 30%igen Natriummethylatlösung in Methanol zugetropft und anschließend 12 Stunden unter Rückfluß gerührt. Nach dem Abkühlen wird abgesaugt und mit 160 Methanol und 1000 Teilen Wasser gewaschen. Man erhält 80 Teile (96%) der Verbindung der Formel

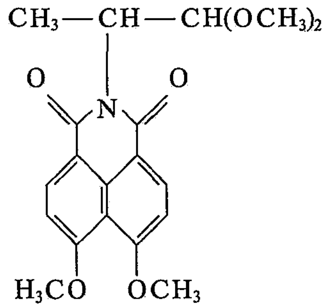

vom Schmelzpunkt 178 bis 179°C.

4,5-Dichlor-N-(1',1'-dimethoxypropyl-2')naphthalimid ist zugänglich durch Umsetzung von 4,5-Dichlornaphthalsäureanhydrid mit 2-Amino-propanaldimethylacetal.

100 Teile des 4,5-Dimethoxy-N-(1',1'-dimethoxypropyl-2')-naphthalimids werden zu einer Lösung aus 680 Teilen N,N-Dimethylformamid, 30 Teilen Wasser und 2 Teilen p-Toluolsulfonsäure gegeben. Die Reaktionsmischung wird 8 Stunden bei 120 bis 130°C gerührt. Das Produkt wird mit 1000 Teilen Wasser ausgefällt, abgesaugt und getrocknet. Man erhält 87 Teile (100%) der Verbindung der Formel

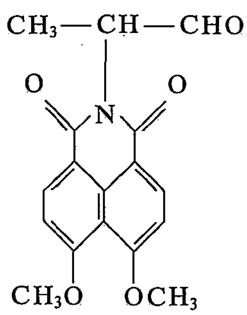

vom Schmelzpunkt 275 bis 278°C (umkristallisiert aus N,N-Dimethylformamid).

Folgende Verbindungen wurden analog dargestellt:

| Bsp. | $R^1$ | $R^2$ | $R^3$ | $R^4$ | Fp. (°C) | Ausbeute (%) |
|---|---|---|---|---|---|---|
| 2 | $CH_3-$ | $-CHO$ | $-C_2H_5$ | $-C_2H_5$ | 261–63 | 91 |
| 3 | $CH_3-$ | $-CHO$ | $-C_4H_9$ | $-C_4H_9$ | 165–67 | 92 |
| 4 | $CH_3-$ | $-CHO$ | $-C_2H_4OH$ | $-C_2H_4OH$ | 242–45 | 98 |
| 5 | $CH_3-$ | $-CHO$ | $-C_2H_4OC_2H_5$ | $-C_2H_4OC_2H_5$ | 188–90 | 92 |
| 6 | $CH_3-$ | $-CHO$ | 70% $CH_3-$<br>30% $C_2H_5-$ | 70% $CH_3-$<br>30% $C_2H_5-$ | 233–35 | 100 |
| 7 | $C_2H_5-$ | $-CHO$ | $-CH_3$ | $-CH_3$ | 262–64 | 90 |

**Patentansprüche**

1. Naphthalimidderivate der allgemeinen Formel

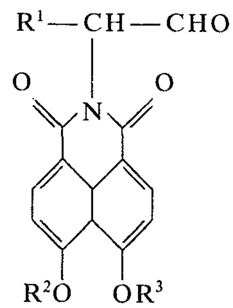

in der

$R^1$ $C_1$- bis $C_4$-Alkyl und
$R^2$ und $R^3$ unabhängig voneinander $C_1$- bis $C_4$-Alkyl, β-Hydroxyäthyl, β-Hydroxypropyl, β-$C_1$- bis $C_4$-Alkoxyäthyl oder β-$C_1$- bis $C_4$-Alkoxypropyl

sind.

2. Verbindungen gemäß Anspruch 1, wobei $R^2$ und $R^3$ gleich oder verschieden und Methyl oder Äthyl sind und $R^1$ die angegebene Bedeutung hat.

3. Die Verbindungen gemäß Anspruch 2 der Formel

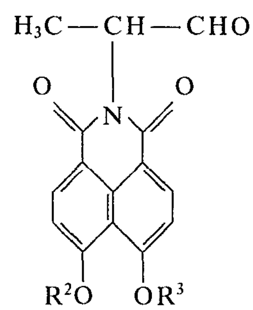

4. Die Verwendung der Verbindungen gemäß Anspruch 1 als optische Aufheller.

**Claims**

1. Naphthalimide derivatives of the general formula

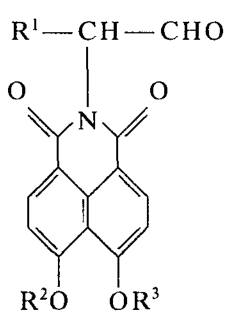

where

$R^1$ is $C_1$- to $C_4$-alkyl, and
$R^2$ and $R^3$ independently of one another are $C_1$- to $C_4$-alkyl, β-hydroxyethyl, β-hydroxypropyl, β-$C_1$- to $C_4$-alkoxyethyl or β-$C_1$- to $C_4$-alkoxypropyl.

2. Compounds as claimed in claim 1, where $R^2$ and $R^3$ are identical or different and are methyl or ethyl and $R^1$ has the stated meaning.

3. Compounds as claimed in claim 2, of the formula

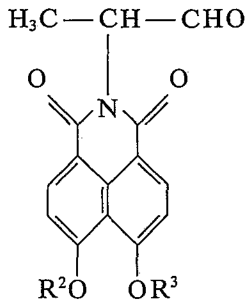

4. The use of compounds as claimed in claim 1 as optical brighteners.

**Revendications**

1. Dérivés du naphtalimide, de formule générale

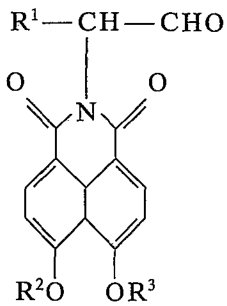

dans laquelle

$R^1$ représente un groupe alkyle en $C_1-C_4$, et
$R^2$ et $R^3$ représentent chacun, indépendamment l'un de l'autre, un groupe alkyle en $C_1-C_4$, bêta-hydroxyéthyle, bêta-hydroxypropyle, bêta-(alcoxy en $C_1-C_4$)-éthyle ou bêta-(alcoxy en $C_1-C_4$)-propyle.

2. Composés selon la revendication 1 dans lesquels $R^2$ et $R^3$, identiques ou différents, sont des groupes méthyle ou éthyle, et $R^1$ a la signification indiquée.
3. Les composés selon la revendication 2, de formule

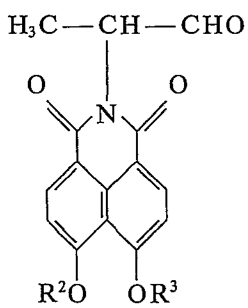

4. L'utilisation des composés selon la revendication 1 en tant qu'azureurs optiques.